# EUROPEAN PATENT SPECIFICATION

(11) **EP 0 696 181 B1**
(45) Date of publication and mention of the grant of the patent: **12.03.2003**
(21) Application number: 94915911.5
(22) Date of filing: 29.04.1994
(51) Int. Cl.: A61B 18/12

(54) **IMPEDANCE FEEDBACK ELECTROSURGICAL SYSTEM**
ELEKTROCHIRURGISCHES IMPEDANZRÜCKKOPPLUNGSSYSTEM
SYSTEME ELECTROCHIRURGICAL A RETROACTION DE L'IMPEDANCE

(30) Priority: 30.04.1993 US 55827
(43) Date of publication of application: 14.02.1996
(73) Proprietor: MEDICAL SCIENTIFIC, INC., Taunton, MA 02780 (US); ETHICON ENDO-SURGERY, Cincinnati, Ohio 45242-2839 (US)
(72) Inventor: NARDELLA, Paul, C., Wareham, MA 02571 (US); YATES, David, Carlyle, West Chester, OH 45069 (US)
(74) Representative: Casey, Lindsay Joseph
(86) International application number: US9404632
(87) International publication number: WO94024949

(56) References cited:
- EP-A- 0 640 317
- WO-A-93/20747
- GB-A- 2 213 381
- US-A- 3 601 126
- US-A- 4 862 889
- US-A- 5 167 658
- US-A- 5 207 691

## Description

### Background of the Invention

The present invention relates to electrosurgical tools which are adapted to deliver electrosurgical energy to tissue.

Surgical procedures often require incisions to be made in human tissue. Such procedures typically require the application of force to a surgical tool having one or more sharp, tissue-contacting edges, and usually create bleeding at the site of the incision. The ease with which the tool makes the incision and the prompt control or elimination of the bleeding thereby created is of paramount importance to the success and safety of the procedure.

Currently known surgical cutting devices utilize different techniques to make incisions and to control or eliminate bleeding. One known device is the Proximate Linear Cutter available from the Ethicon, Inc. of Cincinnati, Ohio. This device is specifically adapted to make an incision in tissue or an organ such as the intestine. The device engages a portion of the tissue or organ between two tyne-like members. To effect cutting, a blade mounted on one of the tynes travels along a predetermined path, thereby making a linear incision through the tissue or organ. Surgical staples are deployed by the cutting device on either side of the incision, resulting in the separation of the organ into two segments, each of which is sealed adjacent to the incision by surgical staples. Despite the use of surgical staples and the precise cutting of the tissue, bleeding is not entirely eliminated and separate cauterization procedures must often be utilized to control or stop bleeding.

Surgical devices also are known which utilize electrical current in the form of radio frequency (RF) energy to incise and to cauterize tissue to control bleeding. U.S. Patent No. 4,651,734 discloses a surgical scalpel modified to include an electrode. This scalpel has the ability to cut tissue and, when properly positioned, to cauterize tissue following a cutting procedure. Such a surgical tool is useful but does not simultaneously cut and cauterize tissue. The separate cauterization procedure which must be utilized is relatively time consuming and may result in unnecessary bleeding. Moreover, such a scalpel is not well suited to many surgical procedures such as the transection of the intestine.

Because of this, the tool must carefully controlled during surgery to ensure that the correct amount of RF energy is applied to the target tissue. For example, if a surgical tool delivers RF energy through a cutting edge to tissue at a magnitude sufficient to cut or cauterize tissue, tissue burns could result if the cutting edge contacts the tissue for too long a period. Similarly, if the cutting edge is moved too quickly through tissue, the optimal amount of energy may not be applied to the tissue. Thus, if not used properly, currently known electrosurgical tools may not take full advantage of the benefits of electrosurgery.

Paul C. Nardella, in "Radio Frequency and Impedance Feedback", SPIE, vol. 1068, p. 42-48, 1989, discloses a study on the potential use of tissue electrical impedance as a feedback parameter for the control of therapeutic radio frequency energy in recanalization and ablation procedures.

United States Patent Specification No. A-4,969,885 discloses an electro-surgery device for cutting and/or coagulating biological tissue using high frequency AC energy. The device includes an active electrode 13 and a neutral electrode 14 which are in circuit with an electronically controlled power supply 16. The circuit has a regulation lop that relays output voltage value to the power supply which then controls the operating voltage of the device before and during use to effect output voltage.

United States Patent Specification No. A 4,474,179 discloses an apparatus for the high frequency coagulation of protein having an impedance measuring means 10 for measuring protein impedance. A differentiator 11 is attached to the impedance measuring means 10 to calculate the differential quotient of the tissue impedance, which value is then used to control the power output of the apparatus. A monitor 17 is also connected to the differentiator 11 and provides acoustic indication of variance in the differential quotient.

Accordingly, an object of this invention is to provide an electrosurgical system which enables electrosurgical tools to conveniently and safely incise and/or penetrate human tissue with controlled and precise application of RF energy. It is another object of the invention to provide a surgical tool which has improved cutting capability and which decreases some of the risk associated with surgery by minimizing the amount of bleeding resulting from incisions and tissue penetration. Another object is to provide a surgical tool which is adapted to simultaneously cut and cauterize tissue. It is also an object to provide bipolar and/or monopolar electrosurgical systems that provide visual, audible, or tactile feedback to a user concerning tissue condition. Other objects of the invention will be apparent upon reading the disclosure which follows.

### Summary of the Invention

The invention attains the aforementioned objects by providing a system which measures tissue impedance as a feedback parameter from surgical tools that apply electrosurgical energy to tissue during surgery. The system is useful with a variety of electrosurgical devices including cutting tools and surgical clip applying devices. The system is particularly useful to provide bipolar and/or monopolar electrosurgical tools with feedback capability (e.g., visual, audible or tactile) which relates, in part, to the impedance targeted within the tissue.

In one aspect, an impedance feedback electrosurgical system constructed in accordance with the invention includes an electrosurgical tool which is adapted to receive power from an electrosurgical power source and to deliver electrosurgical energy to tissue through an active, energy delivering electrode in contact with the tissue. A return or second electrode is also attached to the tissue and is electrically insulated from the active electrode. Electrosurgical energy is thus communicated from the active, energy delivering electrode, through the tissue, and to the return electrode.

Preferably, an impedance monitoring device is in circuit with the active and return electrodes to measure the impedance of the target tissue, which can be ascertained based upon the voltage and current of the applied energy. A power control module is also in circuit with the impedance monitoring device to regulate the electrosurgical energy delivered to the tissue through the active electrode by responding to a signal representative of tissue impedance derived by the impedance monitor. The system is able to control the electrosurgical energy applied to the tissue by monitoring and controlling tissue impedance to within a preselected range. The operating range of the electrosurgical system is preferably between about 20 to 1000 Ohms. This range can even extend to an upper limit of 2000 ohms in some instances. The preselected control range of desired tissue impedance is a narrower range within this operating range. This preselected control range is selectable by a user of the system and will depend upon several factors, such as electrode size and character, the electrosurgical tool being used, and tissue type including percent body fat.

In other aspects, the impedance feedback electrosurgical system has control circuitry to enable one to select the control range within which an acceptable measured tissue impedance is maintained during surgery. One or more feedback or warning devices are preferably connected in circuit with the system to warn a user (e.g., by an audible alarm) that measured impedance is outside the preselected control range and to inform the user of the measured impedance.

In yet another aspect, the impedance feedback electrosurgical system regulates the energy delivered to the tissue by varying the applied voltage and/or current of the electrosurgical power source.

In still another aspect, the impedance feedback electrosurgical system controls the electrosurgical energy delivered through the delivery electrode to the tissue by an activation system. An operator can inhibit or transmit the electrosurgical energy delivered to the tissue by selectively operating the activation system, which operates much like an electrical switch.

The advantages of the electrosurgical system of the invention are several. First and foremost, by maintaining a preselected control range of tissue impedance, electrosurgical energy is applied to the target tissue at desirable levels independent of the speed and operation of the tool as used by an operator or surgeon. Furthermore, when an electrosurgical tool is adapted to a system constructed in accordance with the invention, the system will inherently monitor the passage of the tool's active electrode through different tissue types and certain tissue barriers. For example, once the tool penetrates the abdominal wall, the current density at the active electrode will increase in the area where the tissue contacts the electrode. As a result, the circuitry of the electrosurgical system will lower the applied electrosurgical energy to protect that tissue from receiving excessive or unwanted RF energy.

The electrosurgical energy applied to an active electrode in accordance with the invention also improves the mechanical cutting ability of the tool, and more importantly, facilitates the cauterization and/or fusion of the tissue following the incision. The application of radio frequency energy, for example, to the tissue allows the simultaneous cutting and cauterization of the tissue with an effective consistency independent of a user's technique. Moreover, the use of electrosurgical energy to penetrate tissue can eliminate the need for a conventional, sharpened cutting blade. A conductive electrode can deliver sufficient levels of electrosurgical energy to tissue to effectively incise tissue. The electrosurgical energy also enhances other electrosurgical tools such as clip applying devices,

A method for controlling the level of electrosurgical energy applied to tissue by an electrosurgical tool is also provided. Thus, in one aspect, a method is provided for determining at least one of several properties of the system, such as (i) the time-rate derivative of the signal representative of impedance, (ii) the time-average of the voltage, (iii) the time-average of the current, (iv) the time-average of impedance, (v) current which exceeds a predetermined threshold, and (vi) voltage which exceeds a predetermined threshold. These properties are processed, according to another aspect, to evaluate the electrosurgical effects within the tissue so that a logical indicator, e.g., a light, sound, or tactile source, can be activated to inform or warn the user of the evaluated effects.

These and other aspects of the invention are evident in the description which follows and in the accompanying drawings.

### Brief Description of the Drawings

FIGURE 1 schematically illustrates an impedance feedback electrosurgical system constructed in accordance with the invention and in use with an energy supply source and an electrosurgical tool.
FIGURE 2 schematically illustrates an electrical circuit which can be used to measure tissue impedance.
FIGURE 2A shows a graph illustrating typical tissue impedance as a function of the duration of applied electrosurgical energy.
FIGURE 3 schematically illustrates a surgical cutting tool employed in an impedance feedback electrosurgical system according to the invention, including a supply source of electrosurgical energy.
FIGURE 4 is an exploded side view of the electrosurgical cutting tool illustrated in FIGURE 3.
FIGURE 5 is a sectional view of the electrosurgical tools of FIGURE 4 at lines A-A.
FIGURE 6 is a sectional view of the electrosurgical tool of FIGURE 4 at lines B-B.
FIGURE 7 is a sectional view of the electrosurgical tool of FIGURE 4 at lines B-B in an embodiment which does not include a surgical staple cartridge.
FIGURE 8 is a schematic view of an electrosurgical clip application device employed in an impedance feedback electrosurgical system according to the invention.
FIGURE 9 is a side, partially cut-a-way view of the electrosurgical clip applicating device of FIGURE 8.
FIGURE 10 is a schematic view showing a forward, clip deploying portion of a bipolar electrosurgical clip applicating device employed in an impedance feedback electrosurgical system according to the invention.
FIGURES 11A through 11C schematically illustrate the sequence in which a surgical clip is applied using the tool of FIGURE 8.
FIGURE 12 shows the electrosurgical system of FIGURE 1 with further features according to the invention.
FIGURE 12A illustrates a signal conditioning circuit constructed according to the invention and preferably for use with the electrosurgical system of FIGURE 12.

### Detailed Description of the Invention

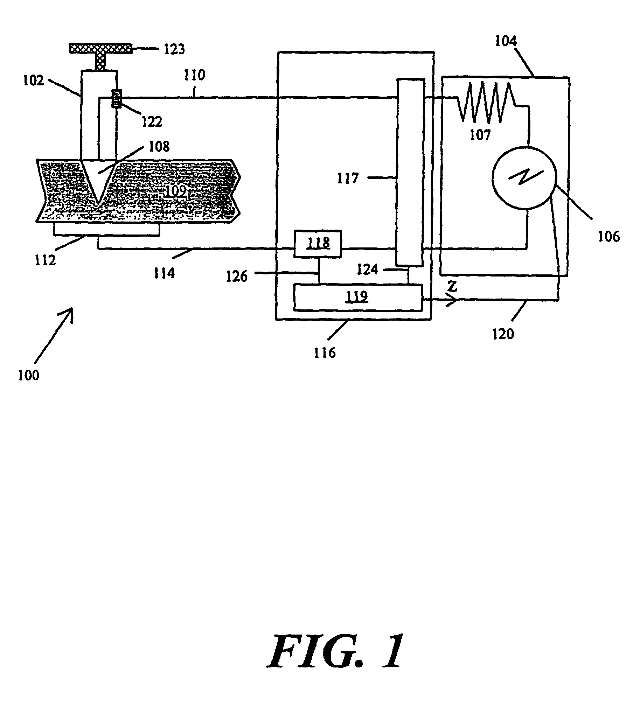

FIGURE 1 illustrates an impedance feedback electrosurgical system 100 constructed according to the invention. The system 100 includes an electrosurgical tool 102 connected in circuit to a power control module 104, shown illustratively with an RF generator 106 and source impedance 107. The tool 102 has an active electrode 108 which operates as a cutting edge and delivers electrosurgical energy to the tissue 109. The active electrode 108 is connected to one terminal of the RF generator 106 through a power supply line 110. A return electrode 112, here shown as a ground pad, is connected to the tissue 109 and to another terminal of the electrosurgical supply 106 via a feedback line 114. An impedance monitor 116 - preferably having a voltage monitor 117, a current monitor 118, and a programmable CPU 119 - connects in circuit with the electrodes 108 and 112 to determine tissue impedance and to generate a signal "z" representative of tissue impedance, which is conveyed to the power control module 104 through a signal line 120. The power control module 104 regulates the electrosurgical energy generated from the RF generator 106 such that the impedance signal "z" remains within a preselected range.

The system preferably includes an activation switch 122 which is operable by a user to selectively generate and control the flow of energy to the active electrode 108 and through the tissue 109.

In operation, force is applied to the tool 102, typically by way of a handle 123, to cause the active electrode 108 to contact the tissue 109 to make incisions and/or to effect cauterization. Electrosurgical energy applied through the tool 102 heats the cells in contact with the active electrode 108 to provide a clean incision. In the course of cutting, the electrosurgical energy applied through the tool 102 also cauterizes tissue to minimize or eliminate any associated bleeding. Without the delivery of electrosurgical energy, e.g., RF energy, through active electrode 108, the surgical incision would be less effective as it would rely solely upon the mechanical sharpness of the cutting blade.

The effectiveness of the cutting and/or cauterization within the tissue 109 is dependent, in part, upon the energy density at the active electrode 108. The effects of electrosurgical energy increase with increased energy density, which is defined as the amount of energy per unit area at the associated electrode. Because the active electrode 108 has a relatively small area as compared to the return electrode 112, its energy density is relatively high, and thus its cauterization or cutting ability is also high. The return electrode 112, on the other hand, has very low energy density at operational currents and thus does not significantly affect the tissue 109.

Regardless of the energy density at either of the electrodes 108 and 112, the current flowing through the tissue and electrodes 108 and 112 is essentially equal. Where a voltage source electrosurgical generator is used, the current is indirectly proportional to impedance.

As noted, tissue impedance is maintained within a preselected control range. Upon delivery of electrosurgical energy to tissue 109 through the active electrode 108, the current and voltage are measured by the current monitor 118 and voltage monitor 117, respectively. These values are used by the CPU 119 to determine impedance, which is the voltage divided by the current. More particularly, voltage is measured by the voltage monitor 117 as a potential difference between the active electrode 108 and return electrode 112; and the current is measured directly through the tissue 109 by the current monitor 118. A signal representative of voltage is transmitted to the CPU 119 via signal line 124. Likewise, a signal representative of current is transmitted to the CPU via the signal line 126. Once voltage and current are known at the CPU 119, impedance is determinable within the tissue 109 and the signal "z" is transmitted to the power control module 104. If necessary, the power control module 104 adjusts its output power to maintain "z" within a preselected range.

The output energy of the power module 104 is adjusted automatically and as necessary during operation of the tool 102 to maintain tissue impedance within a preselected control range. For example, when the tool 102 first contacts the tissue, very little area of the electrode 108 is in contact with the tissue, and thus the current through the tissue 109 is low. As more tissue comes into contact with the active electrode 108, the current through the tissue 109 increases. During this transition, the impedance monitor 116 measures the impedance and conveys a signal "z" representative of the measured impedance to the control module 104, which in turn makes any necessary increase or decrease in the electrosurgical energy conveyed to the active electrode 108 to maintain tissue impedance within a desired range. Preferably, this electrosurgical energy is in the radio frequency range, and the operating range for monopolar operation is between about 20 and 2000 Ohms, and typically a smaller range (e.g., up to about 500 ohms) for bipolar electrosurgical operation.

Those skilled in the art should recognize that other devices may replace the CPU 119 of FIGURE 1. For example, discrete logic, programmable EPLDs, look-up tables, EPROMs, and analog processing means may be substituted for the CPU 119 to provide substantially similar operation.

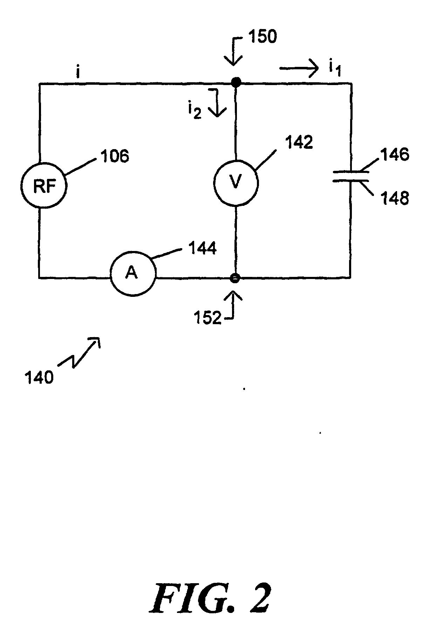

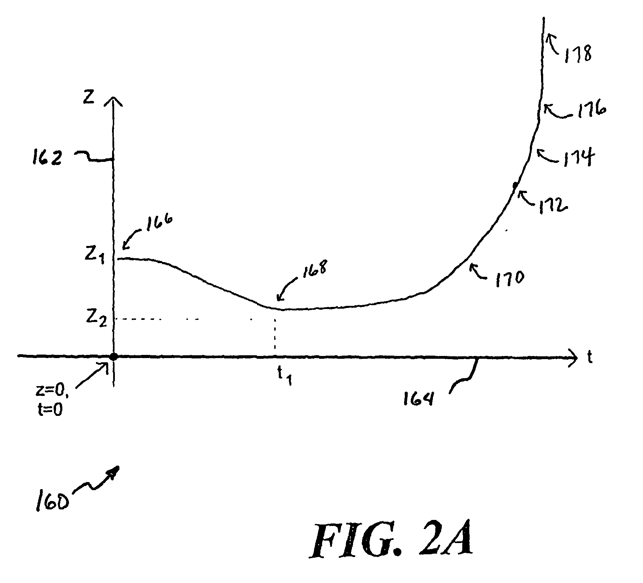

FIGURE 2 illustrates basic circuitry for measuring tissue impedance according to the invention and in a manner that is similar to the operation of the impedance monitor 116 of FIGURE 1. In particular, FIGURE 2 shows a circuit 140 which can be used to determine impedance. The circuit 140 is illustrative and can be altered in any number of ways to function equivalently. Circuit 140 includes an RF generator 106 in circuit with voltage sensor 142, current sensor 144, and electrodes 146 and 148. The voltage and current sensors 142 and 144 are representative of the voltage and current monitors 117 and 118, respectively, of FIGURE 1. One of ordinary skill in the art will readily understand that sensors 142 and 144 can be configured, for example, as (i) transformers or (ii) amplifiers with sensing elements, e.g., resistors. Likewise, the electrodes 146 and 148 are representative of the FIGURE 1 active and return electrodes 108 and 112, respectively. The voltage sensor 142 measures the voltage differential between locations 150 and 152, and the current sensor 144 measures the RF generator current i. FIGURE 2 also shows three representative currents, i, i₁, and i₂, where the current i is the current at a location immediately after the generator 106, and the currents i₁ and i₂ are currents at the respective localities after the split at location 150.

The resistance associated with the voltage sensor 142 is relatively high, i.e., less than about 10% of the current for the entire circuit 140, as compared to the rest of the circuit 140 and thus the current i is approximately equal to the current i₁, which can be measured.

Therefore, provided there is ample wire size, e.g., 22 gauge or larger stranded wire, the voltage differential between localities 150 and 152 is the same as the voltage across the electrodes 146 and 148. Since the current i is known by measuring the current i₁, the resistance between the electrodes 146 and 148 is also determinable. This resistance would be representative of the tissue impedance as described with respect to FIGURE 1. The impedance monitor 116 of FIGURE 1 divides the measured voltage by the measured current to determine tissue impedance. A tissue impedance signal "z" is then generated and transmitted to the power control module of FIGURE 1 (not shown in FIGURE 2) to regulate the applied electrosurgical energy, thereby changing the voltage measured at the voltage sensor 142 and the current measured by the current sensor 144. In this fashion, the measured tissue impedance can be monitored and controlled to within a preselected range.

The measurement of tissue impedance, e.g., corresponding to the resistance measurable in the circuit 140 of FIGURE 2, is readily understood by one of ordinary skill in the art. When the current through the tissue decreases for a given applied voltage, the tissue impedance increases and is readily measured by the impedance monitor 116, shown in FIGURE 1. Tissue cells are most effectively heated and cauterized by RF energy when the impedance is kept to within a preferred electrosurgical range, such as a tissue impedance range within the system's operating range of about 20 to 2000 Ohms. By increasing or decreasing the amount of electrosurgical energy applied to the tissue through the active electrode, tissue impedance is maintained within a preselected control range and the active electrode incises and cauterizes tissue most effectively with less chance of burning tissue. The tissue impedance is a factor of the surface area of the electrodes and distance between electrodes as well as the conductivity of the tissue and the changes in the tissue caused by heating the tissue cells.

The preselected control range of tissue impedance is understood to vary depending upon a number of factors, including the type of tissue subject to electrosurgery, the size of the electrodes, and the design and type of the electrosurgical tool being used. This preselected control range is generally at a portion of an impedance versus time curve shortly after impedance begins to rise rapidly, as described below with respect to FIGURE 2A. Absolute impedance values of the preselected control range will vary based on the factors noted above, but this preferred range generally corresponds to the point just before or just after applied electrosurgical energy causes individual tissue cells to burst. One of ordinary skill in the art will appreciate that the preselected control range of tissue impedance can be predetermined and programmed by the operator of the system, or it can be pre-programmed in a CPU, such as CPU 119 of FIGURE 1, based upon the tissue type undergoing electrosurgery and characteristics of the electrosurgical tool.

FIGURE 2A shows a graph 160 which includes typical data correlating tissue coagulation to impedance and duration of applied electrosurgical energy. The vertical axis 162 represents impedance "z", while the horizontal axis 164 represents time "t", i.e., the duration of contact between the active electrode and the target tissue.

The following description of FIGURE 2A refers to temperature values, within tissue or tissue cells that are typically encountered at different points on the graph shown in FIGURE 2A. It is understood that these temperature values are difficult to ascertain and they should be regarded as estimates. These estimated temperature values may be higher or lower than actual temperature values.

Position 166 represents the initial contact and current flow from the electrosurgical tool through the tissue to the grand pad. By way of example, this initial contact can be represented by the contact of the tool 102 and the ground pad 112 with the tissue 109 as in FIGURE 1. Position 166 thus has a representative tissue impedance z₁, and a tissue temperature of approximately 37°C.

The region of position 168 represents a later time, t₁, and a lower impedance z₂. Accordingly, position 168 illustrates that the impedance drops after applying electrosurgical encrgy to the tissue for a period of time t₁. The temperature of the tissue at position 168 is typically between approximately forty five and seventy degrees centigrade.

As time increases beyond position 168, tissue temperature and impedance remain relatively constant but thereafter begin to rise rapidly. The portion of the curve during which temperature and impedance begin to rise is shown generally by reference numeral 170.

Position 172 represents a location within the preferred preselected control range of tissue impedance. The tissue at position 172 is believed to be approximately seventy degrees centigrade, and has a tissue impedance that is within the system's operating range of 20-2000 ohms. Thus, the electrosurgical system of the invention, such as the system 100 shown in FIGURE 1, can operate to maintain tissue impedance at, slightly above, or slightly below the level corresponding to position 172 of FIGURE 2A. It is believed that the desired range of tissue impedance, which should be approximately the preselected control range of tissue impedance, can be either just before or just after the point at which heating of the tissue causes individual tissue cells to burst.

At impedance values approximately corresponding to position 174, the intracellular fluid is vaporized. This condition is undesirable and the tissue impedance at this level is outside the typical preselected control range of tissue impedance. At even greater impedance values, such as shown by position 176, the tissue temperature is approximately 100°C and the tissue is essentially desiccated. When the tissue temperature reaches approximately 200°C, as illustrated by position 178, carbonization or tissue-charring occurs. The impedance value of charred tissue is quite high and is also beyond the preselected control range.

Thus, in the course of developing the preferred operating realm to most effectively cut and/or coagulate tissue, such as at position 172, the tissue impedance first decreases after the initial contact position 166. Thereafter, more energy is required to offset the increased impedance and to reach position 172. Energy can be regulated upwardly and downwardly, as necessary, to maintain the impedance within the preselected control range of tissue impedance, at impedance values represented by those corresponding to position 172 or impedance values slightly above or below position 172.

It is understood that the graph 160 of FIGURE 2A is representative and is used by way of example only. Minor variations in the shape of the curve may occur depending upon several factors. Factors which may influence the shape and magnitude of the data include: the size of the electrodes as well as the distance separating the electrodes; the type of tissue and in particular the influence of heating or the cellular matter; the design of and materials which form the tool; and the motion of the tool within the tissue. In addition, the responsivity of the tool to generator characteristics and electrosurgical control circuitry may also influence the overall shape of the curve and the magnitude of impedance values. Therefore, the invention can and may operate around a preselected control range of tissue impedance that tissue is somewhat above or below position 172, or which includes position 172.

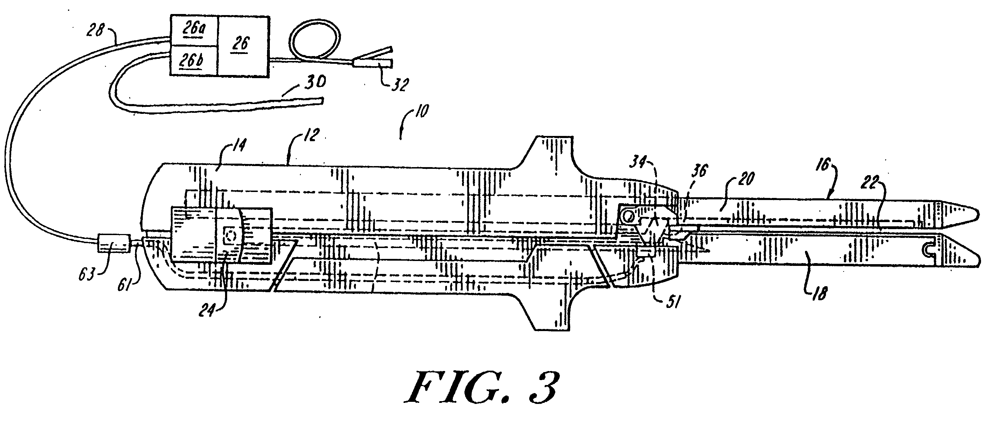

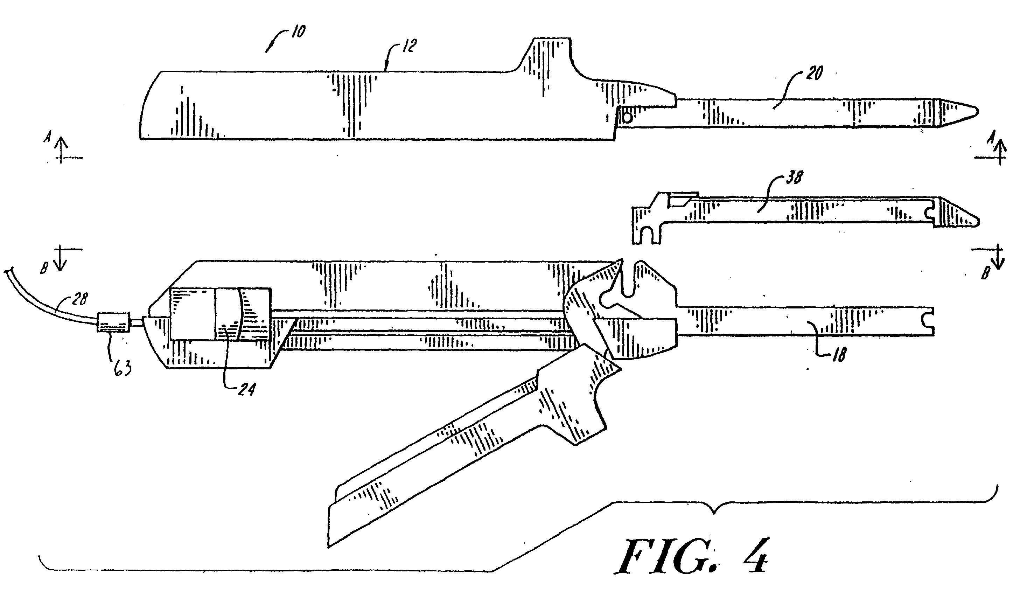

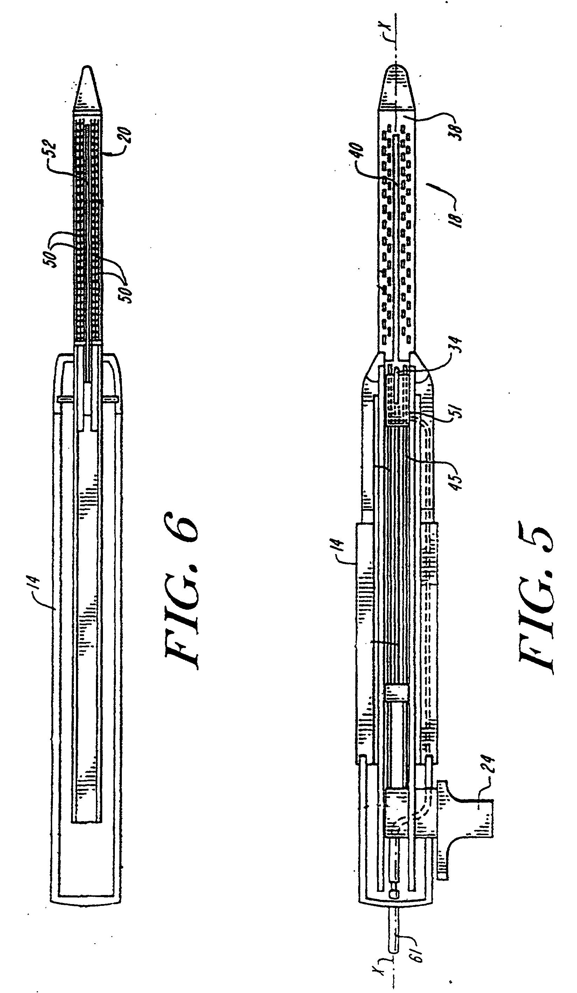

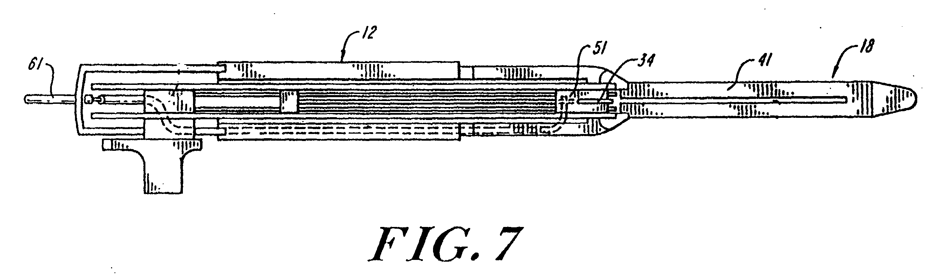

FIGURES 3 through 7 illustrate an embodiment of the invention in which an electrosurgical cutting tool 10 is used with the impedance feedback system of the invention. Cutting tool 10 is a linear cutting tool comprising a housing 12 including a handle portion 14. Adjacent handle portion 14 is cutting template element 16 which includes a first tyne 18 and a second tyne 20. The two tynes 18, 20 of cutting template element 16 are substantially parallel and define a tissue engaging space 22 into which is inserted the tissue or organ to be incised. In a preferred embodiment, the surgical tool 10 includes a lever 24 which facilitates the movement of an active electrode, which may take the form of a cutting blade 34, along a predetermined path.

FIGURE 3 further illustrates an electrosurgical generator 26 which serves as an energy source from which electrical current, preferably in the radio frequency range, is communicated to the cutting tool 10 through insulated wire 28 and connector 63. Insulated return wire 30 communicates to a ground pad (not shown), such as the return electrode 112 of FIGURE 1. A power switch 32, preferably in the form of a foot petal, may be used to break or close the generator 26 circuitry and thus inhibit or transmit the power supplied to the cutting tool 10. Alternatively, a power switch may be disposed on a portion of the cutting tool such as the housing 12.

The circuit representing the power generator 26, the active electrode (e.g., blade 34 with edge 36), wire 28 and delivery wire 61, return wire 30, power control module 26a, and impedance monitor 26b is electrically isolated to control the application of surgical energy by the tool 10. The control module 26a can regulate the electrosurgical energy delivered to the cutting blade 34, according to the measured tissue impedance determined by the impedance monitor 26b. As noted, the impedance monitor generates a signal representative of tissue impedance by quantifying the current through, and voltage differential between; the blade 34 and ground pad (not shown). Current from the source 26 travels through wire 28 and connector 63 and through wire 61, which is electrically attached to the blade 34 by way of connector 51. Current then travels through the tissue and to the ground pad (not shown) for return to the impedance monitor 26b by way of return wire 30. The tissue impedance signal is communicated to the control module 26a which in turn adjusts the applied electrosurgical energy to maintain a measured tissue impedance within a preselected range. Accordingly, electrosurgical power adjustments are made automatically to maintain a skin or tissue impedance to within a safe and operable range, e.g., at approximately the position 172 of FIGURE 2A.

In particular, for radio frequency energy delivered by monopolar generators, the preferred operating range of the system is between 20 and 2000 Ohms. When operating within the preselected control range, which is within the 20 to 2000 Ohm range,, tissue incisions occur with effective cell heating, and further the tissue is cauterized, without burning, to prevent or minimize bleeding and to promote healing.

Although the control module 26a and impedance monitor 26b are shown connected to the power generator 26, it should be apparent that their specific location is irrelevant as long as they permit the simultaneous control and measurement of tissue impedance during the operation of the tool 10. They can thus be easily located on the tool 10.

Blade 34 of tool 10 preferably is retractable when not in use, and moved forward along a cutting path to effect cutting of tissue.

The energy requirements of the electrosurgical tool of the present invention are dynamic and depend to a great extent upon the impedance values of the tissue encountered by the active electrode, e.g., blade 34, during cutting procedures. The impedance of tissue varies among tissue types and the amount of blood present in or around the tissue. The amount of current delivered by the tool to the tissue is a function of the impedance of the tissue. Generally, the amount of current delivered to tissue ranges between about 0.5 and 2.0 amps. The voltage applied to the tissue between the blade and the return electrode, e.g., ground pad, typically is between about 50 to 100 volts rms. These values are typical and are varied automatically to maintain a nearly constant impedance in the tissue during operation of the tool 10.

Surgical tool 10 is particularly well adapted for use in surgical procedures which require transection of an organ such as the intestine. In operation, the tissue (e.g., intestine) is placed within space 22 defined by tynes 18 and 20. The blade is moved forward along the longitudinal axis x of tynes 18 and 20 by movement of lever 24. As the blade moves forward, it passes through the tissue causing it to be severed. Simultaneously, electrical energy, e.g., radio frequency energy, which may be activated for example by foot switch 32, is delivered to the tool and in particular to the blade 34. The electrosurgical current is communicated from the blade 34 to the tissue adjacent the blade and in the vicinity of the incision. Current should be delivered through the blade to the tissue during the entire cutting procedure. A ground pad attached to the tissue communicates the energy back to the monitors 26a and 26b.

The application of electrical energy in this manner is advantageous Electrosurgical energy is delivered through the blade to adjacent tissue to allow for more effective cutting action, and to promote cauterization and/or tissue fusion which effectively eliminates all or substantially all bleeding which results from the incision. The cauterization and/or fusion effect imparted to tissue minimizes blood loss and increases the safety of the surgical procedure as cauterization occurs at substantially the same time that the incision is made.

In a preferred embodiment of the invention, the electrosurgical tool 10 also includes a staple cartridge 38 which houses a supply of surgical staples to be supplied adjacent the incision. The staples may be deployed on one or both sides of the incision to assist in closing the incision and sealing the severed end of the organ. The staples are deployed nearly simultaneously with the cutting action of the blade and the tissue fusion effect imparted by the electrical energy.

One skilled in the art will appreciated that a variety of materials are well suited for the manufacture of the electrosurgical tool 10 shown in FIGURES 2-6. For example, housing 12 and cartridge 38 may be made from or coated with various non-conducting polymers. The conductive components of the tool may be made of various metals, including surgical grade stainless steel and aluminum.

Tool 10 is further described in copending U.S. patent application serial number 019,334, filed February 18, 1993, which is a continuation of serial number 786,572, filed November 1, 1991.

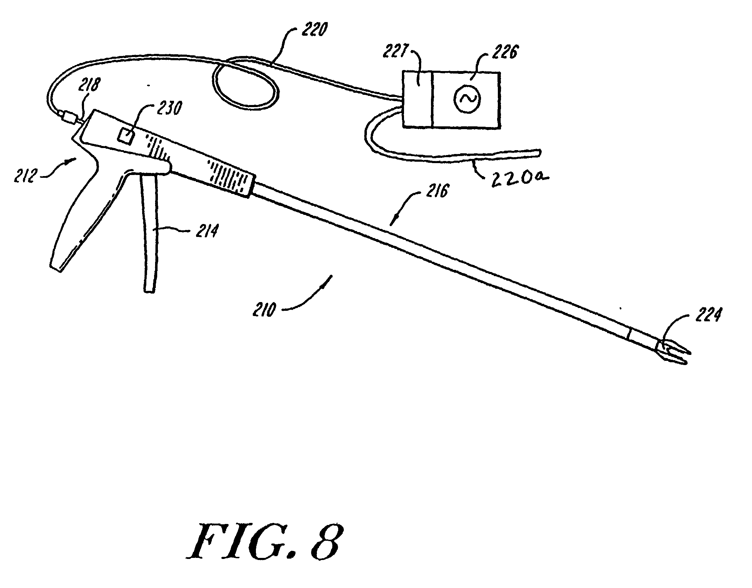

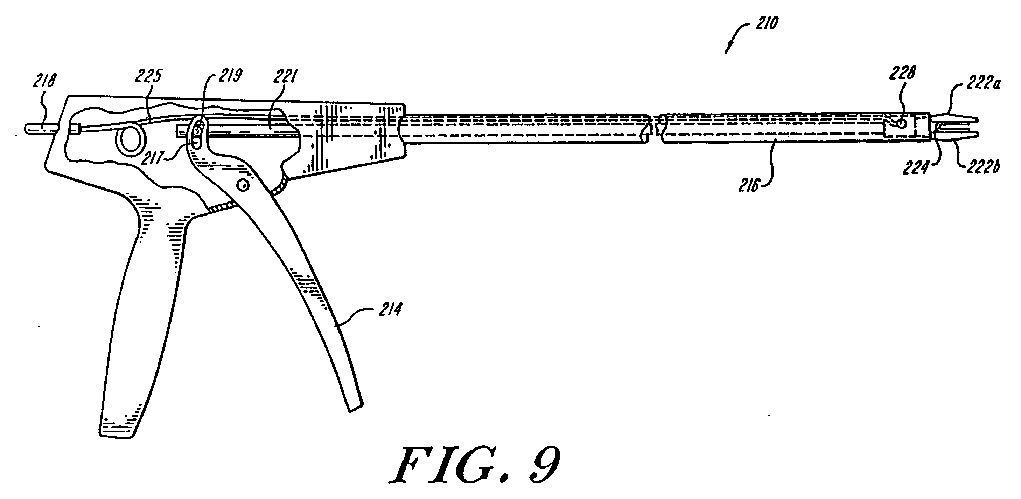

FIGURES 8 through 10 illustrate other electrosurgical tools that may be used with the impedance feedback system of the invention. FIGURES 8 and 9 illustrate an electrosurgical clip applicating device 210 comprising a handle portion 212 having a trigger mechanism 214. Adjacent the handle is an elongate member 216 which houses a supply of surgical clips (not shown) as well as an actuating mechanism, described below, which assists in deploying the clips. The handle 212 also includes an electrical connector port 218 and insulated wire 220, which function to communicate electrosurgical energy to the tool 210 from generator 226.

An actuating mechanism adaptable for use with tool 210 is illustrated in FIGURE 9. The actuating mechanism preferably includes an actuating rod 221 which communicates with the trigger mechanism 214 through a catch 219 which mounts within groovs 217 of trigger 214. Actuating rod 221 also communicates with paired clamping jaws 222a, 222b, which extend from a distal end of barrel 216. The clamping jaws 222a, 222b are adapted to engage and deploy a surgical clip 224. Surgical clips can be deployed by activation of the trigger mechanism 214, causing actuating rod 221 to move backwards (toward the handle 212) while closing clamping jaws 222a, 222b together. When the clamping jaws 222a, 222b are closed, the surgical clip 224 disposed between the jaws is clamped about a duct or vessel. Once a clip is deployed, a new clip may be positioned between clamping jaws 222a, 222b either automatically or manually.

An impedance and power control subsystem 227 functions like the combination of impedance monitor 116 and power control module 118 described in FIGURE 1. Accordingly, subsystem 227 measures the tissue impedance and regulates the power applied to the tool 216 via the active wire 220 and return wire 220a, which connects to the return electrode (not shown) attached to the tissue, e.g., a ground pad.

Although the subsystem 227 is illustrated as located with the generator 226, it is understood that its components and functions can easily be implemented at other locations, most readily with the tool 210.

Electrosurgical generator 226, shown in FIGURE 8, communicates with clipping device 210 through conductive wiring 220 which connects to the clipping device through duplex port 218. As shown in FIGURE 9, port 218 communicates with internally conductive wiring 225 which extends into the clipping device 210. Wire 225 is attached to a conductive portion of the activating mechanism which is in electrical communication with surgical clip 224 to be deployed, thereby functioning as the active electrode. The embodiment illustrated in FIGURE 9 is configured such that the active wire 225 terminates in a connection point 228, which is in electrical communication with the surgical clip 224.

Preferably, the jaws 222a and 222b are non-conductive, as is the barrel 216. In this way, electrosurgical energy is efficiently delivered to tissue and received by the return electrode, such as a ground pad (not shown), through connection with wire 220a, which connects to the negative pole of the power generator 226. With this arrangement, the wires 225, 220 and 220a form an isolated circuit with the generator 226, the impedance and power control subsystem 227, the return electrode attached to the tissue, the active electrode 224, and the tissue when the tool 210 is in operation.

In an alternative embodiment (not illustrated), the active wire 225 may attach to actuating rod 221 which is made from a conductive material and which is in electrical communication with clamping jaws 222a, 222b. The portions of the clipping device 210 which are in electrical communication with active wire 225 (e.g., actuating rod 221 and/or clamping jaws 222a, 222b) preferably are electrically isolated from the remainder of the tool. The return wire of.this configuration is then connected to a separate return electrode (not shown) arranged in contact with the tissue during operation of the tool 210 and communicates with the negative pole of the generator 226. Upon activating the delivery of current to tool 210, for example by activating switch 230, current will be delivered through the wire 225 and communicated to the active electrode, i.e., the surgical clip 224, through actuating rod 221 and/or clamping jaws 222a, 222b. The return electrode receives the electrosurgical energy transmitted through the tissue from the active electrode and electrically connects with the impedance and control subsystem 227 via return wire 220a.

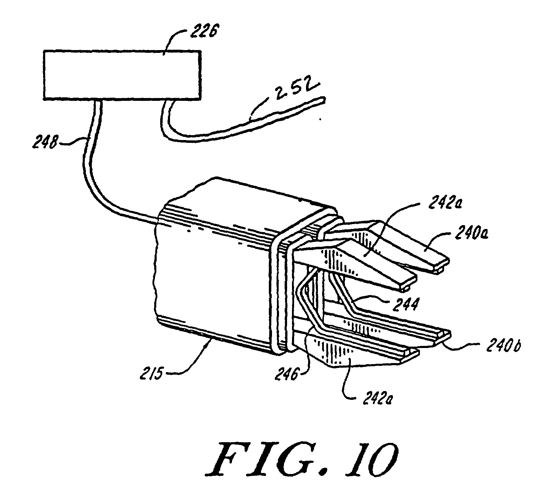

FIGURE 10 illustrates an alternative clip applying tool which can be used with the present invention. Reference numeral 215 represents a forward portion of the barrel 216 which is adapted to receive dual pairs of clamping jaws 240a, 240b and 242a, 242b. The clamping jaws 240a, 240b and 242a, 242b each communicate with their respective actuating mechanisms (not shown). Surgical clips 244 and 246 are shown positioned within jaws 240a, 240b and 242a, 242b.

Insulated wire 248 functions like the active wire 220 shown in FIGURE 8 and communicates electrosurgical energy from generator 226 to clamping jaws 242a, 242b (or, alternatively, to the actuating mechanism associated with clamping jaws 242a, 242b) and jaws 240a, 240b (or, alternatively, to the actuating mechanism associated with jaws 240a, 240b). Upon activation of a trigger mechanism, jaws 240a, 240b and 242a, 242b close together to deploy clips 244 and 246. At the same time a control switch is activated to deliver electrical current to the jaws 240a, 240b, 242a and 242b (or actuating mechanisms associated with these jaws), and hence to clip 244 and 246, which function together as the active electrode. When the clip 244 and 246 contact tissue, current is conveyed to the tissue causing the tissue and clips to be fused together. The electrosurgical energy also promotes tissue-to-tissue fusion. The applied current is returned to generator 226 from the ground pad (not shown) and wire 252.

A generator, impedance monitor and power control module are collectively shown in FIGURE 10 as module 226 and operate in a manner described above to supply electrosurgical energy to the clipping device 210. Virtually any generator able to provide electrosurgical energy for medical applications may be used with the present invention. Preferably, the generator is a voltage determinative, low source impedance generator which provides radio frequency energy. Preferably, a suitable generator can supply up to two amps of current and has a source impedance value of less than 10 ohms. Further details regarding the energy requirements of tool 210 are discussed above with respect to cutting tool 10 above.

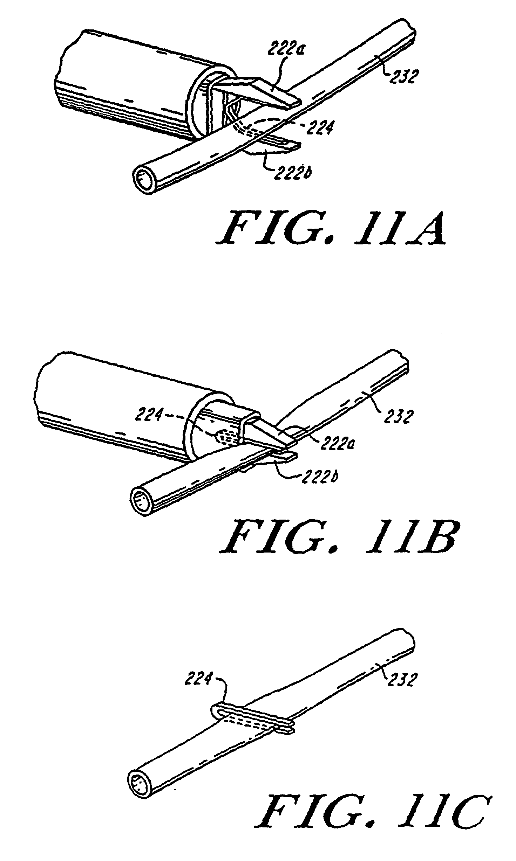

FIGURES 11A, 11B and 11C illustrate the manner in which surgical clips of FIGURES 8-10 are deployed. A vessel 232 to be ligated is disposed between clamping jaws 222a, 222b and surgical clip 224. Upon activating the triggering mechanism, the clamping jaws move together as shown in Figure 11B, causing surgical clip 224 to close upon vessel 232. When the triggering action is completed the clip 224 remains adhered to the vessel 232 as illustrated in FIGURE 11C. While the clip is applied over the vessel, electrosurgical energy is delivered through the clip 224, which functions as the active electrode. Current is maintained for a suitable period of time, usually 5 to 15 seconds, to enable tissue-to-clip and tissue-to-tissue fusion to occur. A return electrode, e.g., a ground pad (not shown), communicates with the generator through a return wire to complete the circuit with the vessel 232.

The activating mechanism of clip activator 210 preferably is made of a conductive material which has a relatively high tensile strength. Exemplary materials include surgical grade stainless steel and aluminum. Clamping jaws 222a, 222b likewise are made of a surgically compatible, conductive material suitable to enable current to be communicated through the clamping jaws 222a, 222b to clip 224. The surgical clips 224 used with the clipping device of the invention may be with a variety of constructions and may be made of variety of conductive, surgically compatible materials, e.g., surgical grade titanium, which are well known in the art. As illustrated the surgical clip may be substantially U- or V-shaped, but various other shapes or constructions are possible as well.

The handle portion 212, trigger 214, and the barrel 216 are electrically isolated from the remainder of the device. Preferably, these components are made of, or are coated with, non-conductive materials such as suitable polymers.

The construction and operation of tool 210 is further described in U.S. Patent No. 5,207,691, issued May 4, 1993.

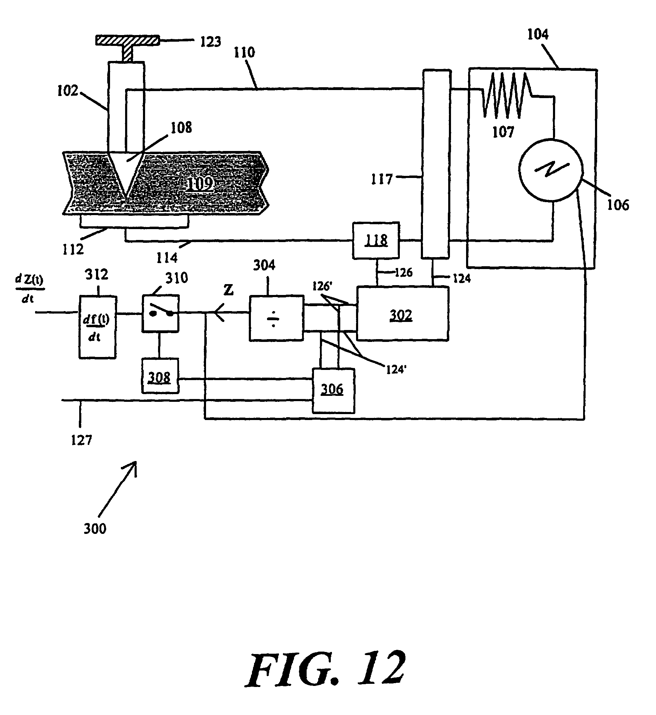

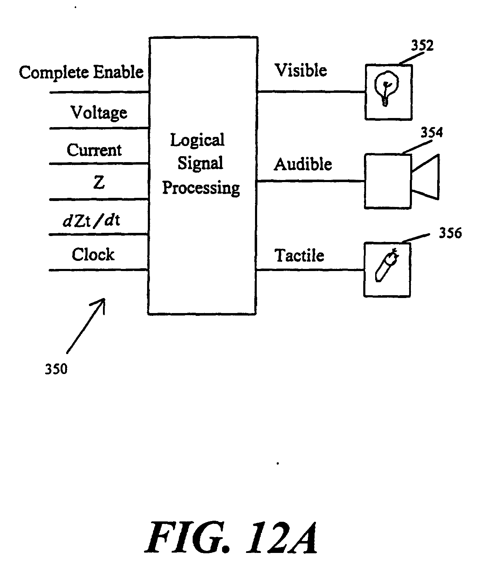

FIGURE 12 shows an electrosurgical impedance feedback system 300 constructed according to the invention which is similar to system 100 of FIGURE 1, but which includes additional features and structure. Feedback system 300 like system 100 shown in FIGURE 1, is shown in contact with tissue 109 and includes RF generator 106, source impedance 107, power control module 104, electrosurgical tool 102, voltage and current monitors 117 and 118, return electrode 112, and feedback line 114. The tool 102 has active electrode 108 and is connected to the module 104 via power supply line 110.

Feedback system 300 differs from system 100 in that it has a signal conditioning module 302, impedance determination module 304, comparator limit module 306, enable derivative module 308, switch module 310, and derivative module 312. These modules operate as follows:

The signal conditioning module 302 conditions voltage and current as received, respectively, from the voltage monitor 117 and current monitor 118 via signal lines 124 and 126. Typically, the signal conditioning module "smoothes" or averages the data from the monitors 117 and 118 by methods known to those skilled in the art, e.g., root-mean square (RMS) techniques, to accurately represent the voltages and currents within the tissue 109. One commonly available integrated circuit which can be used as the module 302 is the LT1088 RMS-dc converter available from Linear Technology of Milpites, California.

RMS can characterize the heating value of the applied electrosurgical energy waveform, thereby averaging the energy delivery to the tissue over a particular and selected time interval. In a preferred embodiment of the invention, this time-averaging is between approximately five and twenty milliseconds.

The impedance determination module 304 communicates with a signal conditioning module 302 via current signal line 126' and voltage signal line 124'. The respective current and voltage signals transmitted to the impedance determination module 304 are thus conditioned or averaged versions of the current and voltage signals transmitted to the signal conditioning module 302 on the lines 126 and 124. The impedance determination module 304 provides a signal "z" which is proportional to the voltage signal divided by the current signal. One commonly available integrated circuit which can be used as the module 304 is the DIV100 Analog Divider available from Burr-Brown Corporation of Tucson, Arizona.

The determination of "z" is preferably averaged over time. For example, z may be calculated by dividing V_{RMS} (the time-averaged voltage signal) by I_{RMS} (the time-averaged current signal).

The comparator limit module 306 selectively generates an enable signal which commands further signal conditioning of the impedance signal "z" after the impedance determination module 304. The enable signal is generated upon command by a user of the system 300 or automatically. Once the enable signal is commanded at module 306, other signal processing activities within the system 300 are enabled. The comparator limit module 306 also determines whether certain threshold values of voltage and/or current are exceeded as relayed by the signal conditioning module 302, which reduces the possibility of false detection caused by transients. According to one embodiment of the invention, current should be greater than one amp, and voltage should be greater than fifty volts.

When selected by the comparator limit module 306, the enable derivative module 308 provides a drive signal to a CMOS switch, or other switching technology known to those skilled in the art, within module 310 so that the impedance "z" signal is transmitted to the derivative module 312.

The derivative module 312 determines the time rate derivative of the signal "z". This time rate derivative signal dZ(t)/dt is used in further signal processing, according to the invention, for controlling the application of electrosurgical energy to the tissue with time-dependency. One acceptable range of derivative values, according to the invention, is from 100Ω/S, which is very slow, to 10,000Ω/S, which is very fast. However, the preferred derivative range useful with the invention is about 200-1000Ω/S.

FIGURE 12A illustrates logic signal processing circuitry 350, e.g., an EPLD, constructed according to the invention and which is preferably used in conjunction with impedance feedback system 300 of FIGURE 12. Circuit 350 assesses certain quantitative aspects of signals relating to the electrosurgical system by combinations of events and signal levels as shown. As illustrated, inputs to the circuit 350 include: the complete enable signal from the comparator limit module 306 via signal line 127; voltage and current signals from the signal conditioning module 302 via signal lines 126' and 124'; impedance "z" from the impedance determination module 304; and the time-rate derivative signal d Z(t)/dt from the derivative module 312. Other signals not associated with the tissue effect, such as a "clock" signal to represent time, may also be input to the logic signal processing circuit 350 to determine or otherwise assess system performance and operation.

These signals are used to activate certain selective logic events. For example, if impedance "z" exceeds a preselected range, any one of selected outputs can be enabled, e.g., a visible source 352, an audible source 354, or a tactile "touch" source 356. Other warnings or indications can also be given. For example, in one embodiment, when the signal "z" is within its preferred and selected range, a green LED is illuminated at the visible source 352; and when "z" is outside the preselected range, a red LED is illuminated. Further, the derivative signal dZ(t)/dt is especially helpful, for example, in signal processing applications as it eliminates certain impedance offsets which can occur at the electrosurgical tool 102 and ground pad locations, as well as within various tissue types.

It is to be understood that the scope of the present invention encompasses electrosurgical tools having constructions other than those specifically described herein. The present invention is potentially applicable to any electrosurgical device utilized in an impedance feedback electrosurgical system according to the invention in which electrosurgical energy is delivered through the device to tissue in contact with the device.

## Claims

1. An impedance feedback electrosurgical system (100) for cutting and/or cauterizing living tissue (109) comprising a bipolar electrosurgical device (102) having a cutting portion including opposed, first and second tissue engaging surfaces (18, 20) defining a tissue engaging space (22) therebetween, wherein one of the first or second surfaces includes an active, energy delivering electrode (108) ;
a return electrode (112) electrically insulated from the active, energy delivering electrode (108) and being adapted to receive electrosurgical energy delivered by the active electrode (108) through living tissue (109) disposed within the tissue engaging space (22) ;
power means (106), in electrical communication with the active and return electrodes, for supplying radio frequency energy to the electrosurgical device (102); and
power control means (104), in circuit with the electrosurgical device (102); **characterised in that** there is provided
an impedance measurement means (116) in electrical communication with the electrosurgical device (102) and the power means (106), the impedance measurement means (116) having a first electrical connection (114) that is coupled to the return electrode (112) for measuring the electrical impedance of the living tissue (109) disposed within the tissue engaging space (22) based on current and voltage applied to the tissue, the impedance measurement means (116) generating a tissue impedance signal ("Z") representative of tissue impedance;
whereby the power control means (104) is in circuit with the impedance measurement means (116) and the power means (106), for regulating the electrosurgical energy delivered to the living tissue (109) disposed within the tissue-engaging space (22) by the active, energy delivering electrode (108) in response to the impedance signal to maintain the tissue impedance within a preselected range.

2. A system (100) as claimed in claim 1 wherein the range of impedance values is between about 20 Ohms and about 500 Ohms.

3. A system (100) as claimed in claim 1 or claim 2 wherein the power control means (104) regulates the energy applied to the active electrode (108) by varying the voltage applied thereto.

4. A system (100) as claimed in any of claims 1-3 further comprising activation means (122) for selectively activating the power means (106) to deliver electrosurgical energy to the active, energy delivering electrode (108).

5. A system (100) as claimed in any of claims 1-4 wherein the electrosurgical device (102) is an electrosurgical cutting device (10) adapted to cut and substantially simultaneously cauterize the tissue (109) disposed in the tissue engaging space (22) located between the active and return electrodes (108, 112).

6. A system (100) as claimed in any of claims 1-5 further including a cutting element (34) associated with one of the first or second members, the cutting element (34) being movable between a non-operative position within one of the first or second members and an operative position.

7. A system (100) as claimed in claim 6 wherein the cutting element (34) is the energy delivering electrode.

8. A system (100) as claimed in any of claims 1-7 wherein the voltage delivered to the electrosurgical device by the power means (106) is between about 50 volts and about 100 volts RMS.

9. A system (100) as claimed in any of claims 1-8 further comprising acoustical warning means (354), responsive to the tissue impedance signal ("Z") generated by the impedance measurement means (116), for informing the user of a change in a measured tissue impedance.

10. A system (100) as claimed in any of claims 1-9 further comprising acoustical warning means (354), responsive to the tissue impedance signal ("Z") generated by the impedance measurement means (116), for alerting a user that the measured tissue impedance is outside of the preselected range.

11. A system (100) as claimed in any of claims 1-10, wherein the active, energy delivering electrode (34) is housed on one of the first or second tissue engaging surfaces (18, 20), and the electrode (34) is movable between an inoperative position within one of the first or second tissue engaging surfaces (18, 20) and an operative, tissue affecting position.

## Patentansprüche

1. Elektrochirurgisches Impedanzfeedbacksystem (100) zum Schneiden und/oder Kauterisieren von lebendem Gewebe (109), umfassend ein bipolares elektrochirurgisches Gerät (102) mit einem Schneidteil, der einander gegenüberliegende erste und zweite Gewebeeingriffsflächen (18, 20) aufweist, die einen Gewebeeingriffsraum (22) dazwischen definieren, wobei entweder die erste oder die zweite Fläche eine aktive Energiezuführungselektrode (108) beinhaltet;
eine Rückführungselektrode (112), die elektrisch von der aktiven Energiezuführungselektrode (108) isoliert und so gestaltet ist, dass sie elektrochirurgische Energie empfängt, die von der aktiven Elektrode (108) durch lebendes Gewebe (109) zugeführt wird, das sich innerhalb des Gewebeeingriffsraums (22) befindet;
eine Energiequelle (106) in elektrischer Kommunikation mit der aktiven und der Rückführungselektrode zum Zuführen von Funkfrequenzenergie zu dem elektrochirurgischen Gerät (102); und
ein Energieregelungsmittel (104), das an das elektrochirurgische Gerät (102) angeschlossen ist,
**dadurch gekennzeichnet, dass** es Folgendes umfasst:
ein Impedanzmessmittel (116) in elektrischer Verbindung mit dem elektrochirurgischen Gerät (102) und der Energiequelle (106), wobei das Impedanzmessmittel (116) eine erste elektrische Verbindung (114) hat, die mit der Rückführungselektrode (112) gekoppelt ist, um die elektrische Impedanz des in dem Gewebeeingriffsraum (22) befindlichen lebenden Gewebes (109) auf der Basis des Stroms und der Spannung in/an dem Gewebe zu messen, wobei das Impedanzmessmittel (116) ein Gewebeimpedanzsignal ("Z") erzeugt, das für die Gewebeimpedanz repräsentativ ist;
wobei das Energieregelungsmittel (104) an dem Impedanzmessmittel (116) und an der Energiequelle (106) angeschlossen ist, um die elektrochirurgische Energie zu regeln, die dem in dem Gewebeeingriffsraum (22) befindlichen lebenden Gewebe (109) von der aktiven Energiezuführungselektrode (108) als Reaktion auf das Impedanzsignal zugeführt wird, um die Gewebeimpedanz in einem vorgewählten Bereich zu halten.

2. System (100) nach Anspruch 1, bei dem der Bereich der Impedanzwerte zwischen etwa 20 Ohm und etwa 500 Ohm liegt.

3. System (100) nach Anspruch 1 oder Anspruch 2, bei dem das Energieregelungsmittel (104) die Energie, die der aktiven Elektrode (108) zugeführt wird, durch Variieren der daran anliegenden Spannung regelt.

4. System (100) nach einem der Ansprüche 1-3, ferner umfassend ein Aktivierungsmittel (122) zum selektiven Aktivieren der Energiequelle (106) zum Zuführen von elektrochirurgischer Energie zu der aktiven Energiezuführungselektrode (108).

5. System (100) nach einem der Ansprüche 1-4, bei dem das elektrochirurgische Gerät (102) ein elektrochirurgisches Schneidgerät (10) ist, dessen Aufgabe es ist, das in dem Gewebeeingriffsraum (22) zwischen der aktiven und der Rückführungselektrode (108, 112) befindliche Gewebe (109) zu schneiden und im Wesentlichen gleichzeitig zu kauterisieren.

6. System (100) nach einem der Ansprüche 1-5, ferner umfassend ein Schneidelement (34), das mit dem ersten oder mit dem zweiten Element assoziiert ist, wobei das Schneidelement (34) zwischen einer Nicht-Betriebsposition innerhalb des ersten oder des zweiten Elementes und einer Betriebsposition beweglich ist.

7. System (100) nach Anspruch 6, bei dem das Schneidelement (34) die Energiezuführungselektrode ist.

8. System (100) nach einem der Ansprüche 1-7, bei dem die von der Energiequelle (106) zu dem elektrochirurgischen Gerät gespeiste Spannung zwischen etwa 50 Volt und etwa 100 Volt (Effektivwert) liegt.

9. System (100) nach einem der Ansprüche 1-8, ferner umfassend ein aktustisches Warnmittel (354), das auf das von dem Impedanzmessmittel (116) erzeugte Gewebeimpedanzsignal ("Z") anspricht und den Benutzer über eine Änderung der gemessenen Gewebeimpedanz informiert.

10. System (100) nach einem der Ansprüche 1-9, ferner umfassend ein aktustisches Warnmittel (354), das auf das von dem Impedanzmessmittel (116) erzeugte Gewebeimpedanzsignal ("Z") anspricht und den Benutzer alarmiert, wenn die gemessene Gewebeimpedanz außerhalb des vorgewählten Bereiches liegt.

11. System (100) nach einem der Ansprüche 1-10, wobei die aktive Energiezuführungselektrode (34) auf der ersten oder der zweiten Gewebeeingriffsfläche (18, 20) untergebracht ist und die Elektrode (34) zwischen einer Nicht-Betriebsposition in der ersten oder der zweiten Gewebeeingriffsfläche (18,20) und einer auf das Gewebe einwirkenden Betriebsposition beweglich ist.

## Revendications

1. Système électrochirurgical à rétroaction d'impédance (100) pour couper et ou cautériser un tissu vivant (109) comprenant un appareil électrochirurgical bipolaire (102) ayant une partie coupante comprenant des première et deuxième surfaces opposées (18, 20) d'engagement avec un tissu, définissant un espace d'engagement de tissu (22) entre celles-ci, où l'une de la première ou de la deuxième surface comprend une électrode active de fourniture d'énergie (108);
une électrode de retour (112) électriquement isolée de l'électrode active de fourniture d'énergie (108), et étant adaptée pour recevoir l'énergie électrochirurgicale fournie par l'électrode active (108) à travers le tissu vivant (109) disposé dans l'espace d'engagement de tissu (22);
un moyen de puissance (106), en communication électrique avec les électrodes active et de retour, pour fournir de l'énergie radiofréquence à l'appareil électrochirurgical (102); et
un moyen de contrôle de puissance (104), en circuit avec l'appareil électrochirurgical (102);
**caractérisé en ce que** le système fournit
un moyen de mesure d'impédance (116) en communication électrique avec l'appareil électrochirurgical (102) et le moyen de puissance (106), le moyen de mesure d'impédance (116) ayant une première connexion électrique (114) qui est couplée à l'électrode de retour (112) pour mesurer l'impédance électrique du tissu vivant (109) disposé dans l'espace d'engagement de tissu (22), sur la base du courant et de la tension appliqués au tissu, le moyen de mesure d'impédance (116) générant un signal d'impédance du tissu ("Z") représentatif de l'impédance du tissu;
en vertu de quoi le moyen de contrôle de puissance (104), est en circuit avec le moyen de mesure d'impédance (116) et le moyen de puissance (106), pour régler l'énergie électrochirurgicale fournie au tissu vivant (109) disposé dans l'espace d'engagement de tissu (22), par l'électrode active de fourniture d'énergie (108), en réponse au signal d'impédance, afin de maintenir l'impédance du tissu dans une plage prédéterminée.

2. Système (100) tel que revendiqué dans la revendication 1, dans lequel la plage des valeurs d'impédance est d'entre environ 20 ohms et environ 500 ohms.

3. Système (100) tel que revendiqué dans la revendication 1 ou la revendication 2, dans lequel le moyen de contrôle de puissance (104) règle l'énergie appliquée à l'électrode active (108) en variant la tension appliquée à celle-ci.

4. Système (100) tel que revendiqué dans l'une quelconque des revendications 1-3, comprenant en outre un moyen d'activation (122) pour activer sélectivement le moyen de puissance (106), afin de fournir de l'énergie électrochirurgicale à l'électrode active de fourniture d'énergie (108).

5. Système (100) tel que revendiqué dans l'une quelconque des revendications 1-4, dans lequel l'appareil électrochirurgical (102) est un appareil électrochirurgical coupant (10), adapté pour couper et cautériser sensiblement simultanément le tissu (109) disposé dans l'espace d'engagement de tissu (22) situé entre les électrodes active et de retour (108, 112).

6. Système (100) tel que revendiqué dans l'une quelconque des revendications 1-5, comprenant en outre un élément coupant (34), associé à l'un des premier ou deuxième membres, l'élément coupant (34) étant déplaçable entre une position non opérationnelle dans l'un des premier ou deuxième membres, et une position opérationnelle.

7. Système (100) tel que revendiqué dans la revendication 6, dans lequel l'élément coupant (34) est l'électrode de fourniture d'énergie.

8. Système (100) tel que revendiqué dans l'une quelconque des revendications 1-7, dans lequel la tension fournie à l'appareil électrochirurgical par le moyen de puissance (106), est d'entre environ 50 volts et environ 100 volts de tension efficace.

9. Système (100) tel que revendiqué dans l'une quelconque des revendications 1-8, comprenant en outre un moyen d'avertissement acoustique (354), sensible au signal d'impédance de tissu ("Z") généré par le moyen de mesure d'impédance (116), pour informer l'utilisateur d'un changement dans une impédance mesurée d'un tissu.

10. Système (100) tel que revendiqué dans l'une quelconque des revendications 1-9, comprenant en outre un moyen d'avertissement acoustique (354), sensible au signal d'impédance de tissu ("Z") généré par le moyen de mesure d'impédance (116), pour informer l'utilisateur que l'impédance mesurée d'un tissu est hors de la plage présélectionnée.

11. Système (100) tel que revendiqué dans l'une quelconque des revendications 1-10, dans lequel l'électrode active de fourniture d'énergie (34) est logée sur l'une des première ou deuxième surfaces d'engagement avec un tissu (18, 20), et l'électrode (34) est déplaçable entre une position non opérationnelle dans l'une des première ou deuxième surfaces d'engagement avec un tissu (18, 20) et une position opérationnelle affectant un tissu.
